# EUROPEAN PATENT SPECIFICATION

(11) **EP 2 340 785 B1**
(45) Date of publication and mention of the grant of the patent: **18.05.2016**
(21) Application number: 10750329.4
(22) Date of filing: 08.02.2010
(51) Int. Cl.: A61F 2/02, A61L 27/20, A61L 27/24, A61L 27/54, A61L 31/06, D01D 5/00, B32B 5/22, A61L 27/14

(54) **ARTIFICIAL DURA MATER AND MANUFACTURING METHOD THEREOF**
KÜNSTLICHE DURA MATER UND HERSTELLUNGSVERFAHREN DAFÜR
DURE-MÈRE ARTIFICIELLE ET SON PROCÉDÉ DE FABRICATION

(30) Priority: 10.03.2009 CN 200910037736; 10.03.2009 CN 200910037739; 22.05.2009 CN 200910039687
(43) Date of publication of application: 06.07.2011
(73) Proprietor: Medprin Regenerative Medical Technologies Co., Ltd., Guangzhou, Guangdong 510663 (CN)
(72) Inventor: XU, Tao, Guangzhou Guangdong 510663 (CN); YUAN, Yuyu, Guangzhou Guangdong 510663 (CN)
(74) Representative: Atkinson, Ralph
(86) International application number: PCT/CN2010/070566
(87) International publication number: WO 2010/102533

(56) References cited:
- WO-A2-2004/000915
- CN-A- 1 274 292
- CN-A- 1 317 297
- CN-A- 101 036 802
- CN-A- 101 507 661
- CN-A- 101 559 242
- CN-A- 101 559 327
- US-A1- 2004 037 813
- US-A1- 2007 233 275
- US-A1- 2008 254 091

## Description

The present invention relates to an artificial dura mater for replacement of defective dural tissue. The present invention also relates to a method of preparing such an artificial dura mater.

Dural defect is a common problem following posterior fossa surgery. The open craniocerebral injury (industrial, traffic or war-related), the invasion of tumor, the congenital meninges defect or other cranial diseases can lead to the defect of dura mater. The defect of dura mater should be repaired timely so as to prevent the leakage of cerebrospinal fluid, the encephalocele, and the stress from barometric pressure. Otherwise, it will endanger the patient's life.

Currently, there are a few alternatives to human dura, but the materials used for artificial dura mater can be classified into four types: autologous fascia, allograft, natural or synthetic substance, and xenograft. However, when those materials are applied to the clinic, the unavoidable shortcomings, such as high infection rate, will appear. According to statistics, the infection rate of craniotomy is 4%; the dura mater made of pig's small intestine mucosa gives the infection rate of 3.4%; and the dura mater manufactured by collagen exhibits the infection rate at 3.8%. Because of blood brain barrier, once the intracranial infection occurs, the encephalic plasma concentration of anti-infection drugs hardly reaches the wanted level and the control of infection becomes difficult. Also, the existing artificial dura products do not have the capability of adding the medicines into the meninges. Therefore, the control of infection after operation is often ineffective.

Moreover, the surgery of brain tumor excision is one of common reasons for use of artificial dura mater to repair the defective meninges. It is known more than half of brain tumours cannot be completely removed. Therefore, the chemotherapy is necessary after the surgery. Many chemical compounds bring poison and fail to pass the blood brain barrier, so that the effective concentration of the applied drugs cannot be reached and the chemotherapy effect is diminished.

The existing artificial dural substitute products usually are not able to add the therapeutic drugs of interest. For instance, based on dense structures, the autologous fascia can not be added with drugs naturally, and the allograft or xenograft can be hardly loaded with drugs. However, due to good malleability, the synthetic material based dural substitutes can be readily added with the drugs. On the other hand, not limited to the adding methods, it is also not easy to incorporate and fix drugs on the artificial dura mater, and to control the release of these drugs as pre-designed. All of these can significantly affect the object of therapy. To date, the common way to add the anti-infection drug is to soak the product with the drug. Under the method, the majority of medicine stays on the surface of artificial dura mater, being easy to flow away, and unable to reach the purpose of controlled release.

In conclusion, the existing artificial dura mater substitutes have shortcomings such as high infection rate, poor bio-compatibility, incomplete absorbability, and difficulty in adding medicine and controlling the effective release of medicine.

A multi-layered anti-adhesion barrier is disclosed in United States patent publication US 2008/0254091 A1, which has a hydrophobic electrospun base layer with a hydrophilic polymer coating layer. The hydrophilic layer adheres the anti-adhesion barrier to a wound, and the hydrophobic layer blocks the infiltration of blood and cells into the wound.

According to a first aspect of the present invention, there is provided an artificial dura mater for use in repair of defective dural tissue as set out in claim 1.

The artificial dura mater according to the invention is made of electrospun layers. The said electrospun layers include at least one hydrophobic electrospun layer, which is manufactured by a kind or kinds of hydrophobic polymers by means of electrospinning technology. The said hydrophobic polymer is selected from the group of hydrophobic aliphatic polyester, polyetherester, polyorthoester, polyurethane, polyanhydride, polyphosphazene, and poly amino acid, and the copolymer or mixture thereof.

Also, the hydrophobic aliphatic polyester is selected from at least one of the group: polylactic acid, polyglycolide, polycaprolactone, and polyhydroxybutyrate (PHB). The said polyetherester is selected from at least one of the group: the polydioxanone (PDO), glycol-lactic acid copolymer, or glycol/terephthalic acid-polybutylece terephthalate copolymer. The said polyanhydride is polysebacic polyanhydride (PSPA)-cetane diacid anhydride.

The artificial dura mater, containing the hydrophobic layer, has the strength similar to that of human dura mater. It can seal brain and prevent the leakage of cerebrospinal fluid before the human's own dura mater recovers or repairs. The hydrophobic layer is not favorable for the cell's migration and attachment, and as a result, the object of anti-adhesion can be obtained. In practice, more than one hydrophobic layer can be set, so as to meet different need for strength.

Furthermore, in the artificial dura mater according to the invention, at least one hydrophilic electrospun layer can be further provided on the said hydrophobic electrospun layer. The said hydrophilic layer is made of one kind or more kinds of hydrophilic polymers in the electrospinning way. The said hydrophilic polymer is selected from the group consists of chondroitine sulfate, heparin, agar, glucan, algin, cellulose, modified cellulose, sodium alginate, starch, gelatin, fibrinogen, silk protein, elastin-mimicry peptide polymer, collagen, chitosan, modified chitosan, hydrophilic polyurethane, polyethylene glycol, polymethylmethacrylate, PMMA, PHBV, PHBHHx, polyvinyl alcohol, or polylactide, and the mixture of two or more aforesaid materials.

When the dura mater is transplanted into brain, the hydrophobic layer is set close to the brain surface and functions to prevent adhesion, while the hydrophilic layer is far to the brain and provides fine nanofibers based cytoskeleton for the adhesion, migration, proliferation, and differentiation of cells. As the hydrophilic layer is made of the hydrophilic material that exhibits good bio-compatibility, it can effectively induce the migration and proliferation of stem cells and fibroblasts, and consequently promote the growth of autologous dura mater. In practice, the hydrophilic layer can be more than one layer, so as to satisfy different needs to be achieved.

According to one embodiment of the invention, the inventive dura mater can also have a transition layer between the hydrophobic and hydrophilic layers. The transition layer is made of one or more kinds of polymers in the electrospinning way. The affinity of transition layers to water are increased from the hydrophobic layer to the hydrophilic layer. With this transition layers, the affinity between the hydrophobic layer and hydrophilic layer can be improved.

According to the other embodiment of the invention, each of the hydrophobic layers, the hydrophilic layers, and the transition layers can be added with cytokine and/or the medicine. The composite layers, once transplanted into the brain, can release the cytokine and/or the medicine into the brain, along as the polymer is degraded. The cytokine and/or the medicine then can function to prevent the local infection, adhesion, and/or tumor's recurrence, or promote the restoration of autologous dura mater.

According to another embodiment of the invention, the hydrophobic layer and the hydrophilic layer can be added with the cytokine and/or the medicine.

The cytokine mentioned in the invention is the factor that works for the adhesion, migration, proliferation, and differentiation of fibroblast, including the interleukin, colony stimulating factor, tumor necrosis factor, platelet derived growth factor, basic fibroblast factor, or the compound of two or more foresaid factors. The cytokine can speed up the recovery process of the defective dura mater.

The medicine stated in the invention is one or more among antibiotic, hemostat, anti-adhesion agent, and tumor-resistance drug. Those medicines are placed onto the artificial dura mater in accordance with the actual need. Once the artificial dura mater is transplanted, the medicines are released gradually to achieve therapeutic purpose during the process in which the polymer is degraded and the dural defect is being repaired. In this way, the blockage from blood brain barrier to medicine is overcome.

What's more is that the cytokine and/or medicine can be encapsulated within the hydrogel. With the adhesion and fixation functions of hydrogel, the medicine can, in line with the local situation, be equally or specially released. The said hydrogell can be selected from one or more substances of polysaccharide polymer, polypeptide polymer, or synthetic hydrophilic high molecular polymer.

According to one embodiment of the invention, the hydrophobic electrospun layer of the said artificial dura mater has a fiber diameter of 50-1000nM. According to one embodiment of the invention, the pore size of hydrophobic electrospun layer is less than 3µM, while the hydrophilic electrospun layer has a fiber diameter of 5-200µM with a pore size of 20-200µM. The pore size depends largely on the diameter of fiber. When the diameter of fiber decreases, the pore size reduces. So, the control of fiber's diameter can realize the control of pore size of electrospun layer. The average diameter of human cells is 10-50µM. The meninges mainly consist of the fibroblasts and collagen fibers excreted from fibroblast. Most of fibroblasts have the diameter between 20 and 30 µM. Where the pore size of the hydro is set at less than 3µM, it can prevent the entry of cell, and prevent the adhesion of dural mater to brain tissue. Where the hydrophilic layer has the pore size equal or larger than the average diameter of cell, it can promote the migration and entry of cell.

According to a second aspect of the present invention, there is provided a method of preparing the aforesaid artificial dura mater for use in repair of defective dural tissue comprising the steps set out in claim 13.

Further, the said hydrophobic aliphatic polyester can be selected from at least one of the following group consists of polylactic acid, polyglycolide, polycaprolactone, and polyhydroxybutyrate. The said polyetherester can be selected from at least one of the following group consists of polydioxanone (PDO), glycol/lactic acid copolymer, and glycol/ polybutylece terephthalate copolymer. The said polyanhydride can be polysebacic polyanhydride-cetane diacid anhydride.

The method is to apply the principle of electrospinning to form the artificial dura mater by using the particular polymer. The dura mater can effectively prevent its adhesion to brain tissue.

To further prevent the adhesion of artificial dura mater to brain tissue, the fiber's diameter is controlled, and consequently the pore size of the scaffold is under control. In this way, the cell's migration can be stopped. The fiber diameter of hydrophobic electrospun layer is controlled within 50-1000nM and the pore size of hydrophobic electrospun layer is less than 3µM.

In the foresaid method, the procedure B) has the process parameters set in this way. The velocity of micro-injection pump is 0.1-5.0 ml per hour. The voltage of high voltage generator is 5-40KV. The receiving distance is 5.0-30.0 cm.

To achieve the better effect in clinical therapy, the invention also provides the method similar to the above-said method for manufacturing hydrophilic layer. The procedures are A) Put the hydrophilic polymer into solvent and have the electrostatic spinning solution with hydrophilic polymer. The hydrophilic polymer can be selected from the group consists of the following substances: chondroitine sulfate, heparin, agar, glucan, algin, modified cellulose, sodium alginate, starch, cellulose, gelatin, fibrinogen, silk protein, elastin-mimicry peptide polymer, collagen, chitosan, modified chitosan, hydrophilic polyurethane, polyethylene glycol, polymethylmethacrylate, PMMA, PHBV, PHBHHx, polyvinyl alcohol and polylactide. B) Receive the electrospinning solution consisting of hydrophilic polymer on the hydrophobic layer, and obtain the hydrophilic layer.

The hydrophilic electrospun layer is set far to the brain surface, in oder to promote the cell's migration and the regeneration of dura mater. To facilitate the entry of cell, the fiber of hydrophilic electrospun layer should have the diameter of 5-200µM, and the pore size is 20-200µM.

In the formation of hydrophilic electrospun layer, the process parameters in the procedure B) can be set as follows. The velocity of micro-injection pump is 0.1-20.0 ml/h. The voltage of high voltage generator is 10-40KV. The receiving distance is 5.0-30.0 cm.

In the process of electrospinning, generally, the hydrophobic polymer or the hydrophilic polymer should be dissovled into the appropriate solvent so as to form the electrospinning solution. Often, the solvent can be volatile organic one and includes but is not limited to methanoic acid, acetic acid, ethyl alcohol, acetone, dimethyl formamide, dimethyl acetamide, dihydrofuran, dimethyl sulfoxide, hexafluoro isopropyl alcohol, trifluoroethyl alcohol, dichloromethane, trichloromethane, methyl alcohol, ethyl alcohol, chloroform, dioxane, trifluoroethane, trifluoroethyl alcohol, and the mixture of two or more of them. The volatile organic solvents will be quickly volatilized during the process of forming electrospun layers, and the final electrospun layers contain no or little residue of organic solvent which can be removed in the later steps. In some cases, water can be used as the solvent, and should be removed by oven or natural dryness after the electrospun layers are formed.

Furthermore, the manufacturing method according to the invention comprises the step of forming the transition layer between the hydrophobic and hydrophilic electrospun layers by electrospinning before the hydrophilic layer is prepared. The transition layer's affinity to water will be gradually increased from the hydrophilic electrospun layer to the hydrophobic electrospun layer. The materials, solvents, and electrospinning parameters to prepare the transition layer can be determined based on the actual situation and need. The formation of transition layer can increase the affinity between hydrophilic and hydrophobic layers.

In accordance with one embodiment of the invention, during the formation of each electrospun layer by electrostatic spinning, the cytokine and/or medicine can be added to the corresponding solutions. The blending technique enables the layers contain the cytokine and/or medicine, and brings better clinic application and better therapy effect.

The manufacturing method for artificial dura mater according to the invention can further include the step that the cytokine and/or medicine are distributed on the hydrophobic electrospun layer and/or the hydrophilic electrospun layer by the bio-printing technique. In the bio-printing, the hydrophobic electrospun layer and/or the hydrophilic electrospun layer functions as the support/substrate (i.e. bio paper), and the cytokine and/or medicine are printed onto the layers.

To make the cytokine and/or medicine distribute evenly onto the layers, the cytokine and/or medicine can be enclosed into the hydrogel.

Specifically, the bio-printing can be done in following steps. A) Mix the hydrogel solution with the cytokine and/or medicine, and form the mixed solution. B) Apply the bio-printing technology to print the said solution into the hydrophobic and/or hydrophilic layer.

The hydrogel solution in the invention is the aqueous solution with the polysaccharide polymer, polypeptide polymer, or synthetic hydrophilic polymer. The said polysaccharide polymer includes but is not limited to the starch, cellulose, sodium alginate, hyaluronic acid and chitosan. The said polypeptide polymer includes but is not limited to the collagen, poly-L-lysine, and PLGA. The said synthetic hydrophilic polymer includes but is not limited to crylic acid, polymethacrylic acid, polyacrylamide, and N-isopropyl acrylamide.

The hydrogel is in the form of liquid under the normal circumstance. At appropriate temperature or special condition, it can turn to gel for a short time and so it offers good adhesion. In the invention, some types of hydrogel need the cross-linking agent in participation of reaction. Therefore, before the procedure of bio-printing, as a pretreatment process the solution with cross-linking agent can be touched with or added into the hydrophobic and/or hydrophilic layer. And the pretreatment can make the cross-linking agent adhere to the layer or layers. After that, the cytokine and/or medicine will be mixed into the hydrogel solution, and the mixed solution will be placed into the print head. In print, when the hydrogel solution with cytokine and/or medicine contacts the electrospun layers, the solution solidifies and adheres to the layers. The uniform and stable printing at designed speed is adopted, so that the cytokine and/or medicine can be evenly released. Also, the customized printing with varied speeds and locations can be used for the individual case so as the cytokine and/or medicine can be released on specific area. The selection of cross-linking agent is based on the type of hydrogel. When the hydrogel is sodium alginate, the cross-linking agent is calcium chloride. When the hydrogel is fibrinogen, the cross-linking agent is thrombin.

Compared with the prior art, the invention exhibits the following advantages:
(1) Its mechanical properties satisfies the requirement for tensile strength and flexibility, and it is waterproof and anti-adhesion.
(2) The membrane's material is free from poison and harmless to human body, possesses good tissue bio-compatibility, can be completely absorbed after implantation, and can avoid the occurrence of tumor or cancer.
(3) The membrane is not made from animal tissues, therefore it can avoid such risks as the immune rejection, the virus spreading, and disease infectiousness.
(4) The designed double layers can prevent adhesion, promote the growth of autologous cell, and enable the earlier restoration of dural mater.
(5) By incorporating the bio-printing technology, the therapeutic substances are introduced into the membrane, and can be released in a controlled manner after the implantation.
(6) The materials have a wide variety of sources, cost low and are convenient in transportation and store.
(7) The manufacturing process comprises easy procedures, costs low, and is easy to mass production.
(8) The clinical application is simple, and the patient customized application is also available.

The other aspects or advantages of the invention will be further given in the following descriptions, and part of them will become obvious through the following descriptions or be understood more easily through the practice.

The invention will now be described by way of example only, with reference to the accompanying drawings, of which:
Figure 1 is the illustration of electrospinning process to manufacture the artificial dura mater according to the invention;
Figure 2 is the illustration of the process for manufacturing the artificial dura mater according to the invention that combines the electrospinning with the bio-printing;
Figure 3 is the illustration of the artificial dura mater according to the invention consisting of hydrophobic electrospun layer;
Figure 4 is the illustration of the artificial dura mater according to the invention consisting of hydrophobic electrospun layer and hydrophilic electrospun layer;
Figure 5 is the illustration of the artificial dura mater according to the invention consisting of hydrophobic layer, hydrophilic layer and transition layer;
Figure 6A is the illustration of the artificial dura mater according to the invention through blending, which consists of hydrophobic electrospun layer and hydrophilic electrospun layer;
Figure 6B is the magnified illustration of the Zone I of Figure 6A;
Figure 7A is the illustration of artificial dura mater according to the invention through blending, which consists of hydrophobic electrospun layer, hydrophilic electrospun layer and transition layer;
Figure 7B is the enlarged illustration of Zone II of Figure 7A;
Figure 8A is the illustration of artificial dura mater according to the invention obtained with the bio-printing technology;
Figure 8B is the enlarged Zone III in Figure 8A;

In the figures, the numbers represent in this way;
- 1: Electrospinning sprayer;
- 2: Spinning thread;
- 3: High voltage power source;
- 4: Receiving device;
- 5: Bio-printer head;
- 6: Vessel;
- 7: Hydrophobic spinning thread;
- 8: Medicine;
- 9: Hydrophilic spinning thread;
- 10: Cytokine;
- 11: Spinning thread for transition layer;
- A: Hydrophobic electrospun layer;
- B: Hydrophilic electrospun layer;
- C: Transition layer.

### Best Mode for Carrying Out the Invention

Now, examples of the invention will be explained in detail. The illustration of examples will be shown in the accompanying drawings, in which the same number represents the same or similar elements. The examples described with reference to the drawings are only illustrative to intend for interpreting the invention and can not mean the limitation to the invention.

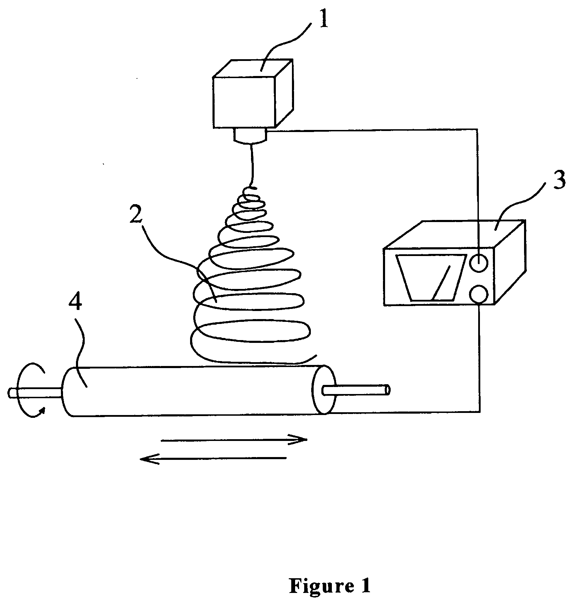

Now, together with Figure 1 to 8, the artificial dura mater according to the invention and its manufacturing method are described.

As shown in Figure 1, the illustration of electrospinning process to manufacture the artificial dura mater, the sprayer 1 for the electrospinning contains the polymer solution and the high voltage power source 3 has its high voltage end connected to the sprayer 1. The receiving device 4 is in cylinder shape, and can be move from left to right, or in reverse way, surrounding the axis of cylinder or the long shaft direction of cylinder. The movement of receiving device 4 can be set with computer program, so that the formed electrospun layer will have the equal thickness. In practice, the receiving device can be set with a level-off surface, and through the movement between left and right or fore-and-aft, the even reception can be realized. The receiving device 4 is connected with the low voltage end of high voltage power source 3, so that there is a larger voltage difference between the sprayer 1 and receiving device 4.

Before the electrospinning starts, the proper polymer solution for electrospinning should be prepared.

It is an option to choose the solution of hydrophobic polymer for electrospinning, and such solution is prepared by dissolving the hydrophobic polymer into the solvent. The hydrophobic polymer includes but is not limited to the hydrophobic aliphatic polyester (covering polylactic acid, polyglycolide, polycaprolactone, and polyhydroxybutyrate), polyetherester (such as polydioxanone), polyorthoester, polyurethane, polyanhydride (such as polysebacic polyanhydride-cetane diacid anhydride), polyphosphazene, polyamino acid and the copolymer or compound of two or more of those polymers.

Depending on the design, if the hydrophilic electrospun layer is needed after the hydrophobic electrospun layer is finished, the solution of hydrophilic polymer for electrospinning should be prepared. The hydrophilic polymer includes but is not limited to the chondroitine sulfate, heparin, agar, glucan, algin, modified cellulose, sodium alginate, starch, cellulose, gelatin, fibrinogen, silk protein, elastin-mimicry peptide polymer, collagen, chitosan, modified chitosan, hydrophilic polyurethane, polyethylene glycol, polymethylmethacrylate, PMMA, PHBV, PHBHHx, polyvinyl alcohol and polylactide. Based on the actual needs, multiple sprayers 1 may be set, in which, the hydrophobic polymer solution or the hydrophilic polymer solution is placed, respectively. Or, the solution in the sprayer 1 is replaced after the hydrophobic layer is made.

The solvent of the solution for electrospinning can be water or the volatile, organic solvent, including but not limited to methanoic acid, acetic acid, ethyl alcohol, acetone, dimethyl formamide, dimethyl acetamide, dimethyl sulfoxide, hexafluoro isopropyl alcohol, trifluoroethyl alcohol, dichloromethane, trichloromethane, methyl alcohol, ethyl alcohol, chloroform, dioxane, trifluoroethane, and trifluoroacetic acid.

Once the solution for electrospinning is ready, the parameters should be set. After that, the power is on, and the electrospinning device is activated. As the spinning thread 2 is drawn from the sprayer 1, the receiving device 4 will move in prescribed procedures, so as to form the uniform electrospun membrane structure.

The parameters for the process to form the hydrophobic electrospun layer are set in this way. The velocity of micro-injection pump is 0.1-5.0 ml per hour. The voltage of high voltage generator is 5-40KV. The receiving distance is 5.0-30.0 cm. The fiber diameter of hydrophobic electrospun layer can be managed between 50 and 1000µM, and the pore size is less than 3µM.

The parameters for the process to form the hydrophilic electrospun layer are set in this way. The velocity of micro-injection pump is 0.1-20.0 ml per hour. The voltage of high voltage generator is 10-45KV. The receiving distance is 5.0-30.0 cm. The fiber diameter of hydrophilic electrospun layer can be managed between 5 and 200µM, and the pore size is from 20 to 200µM.

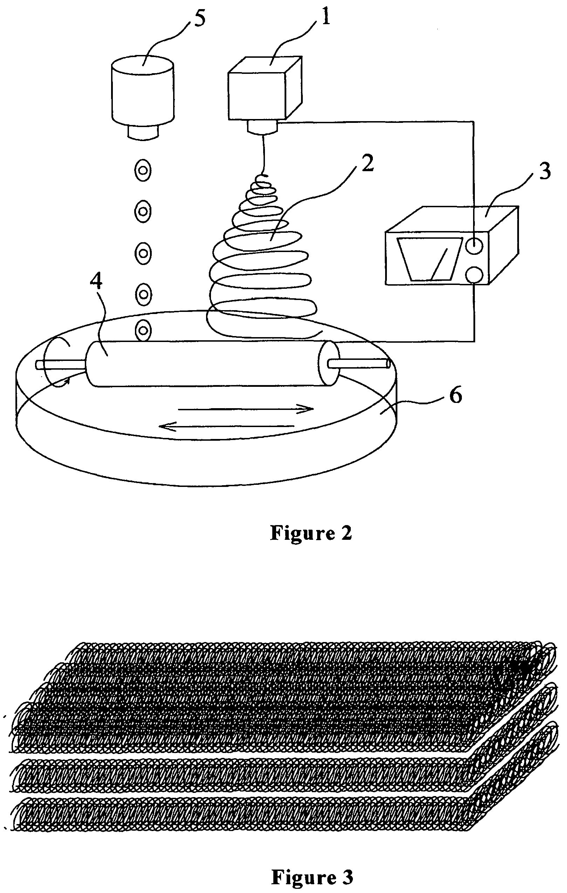

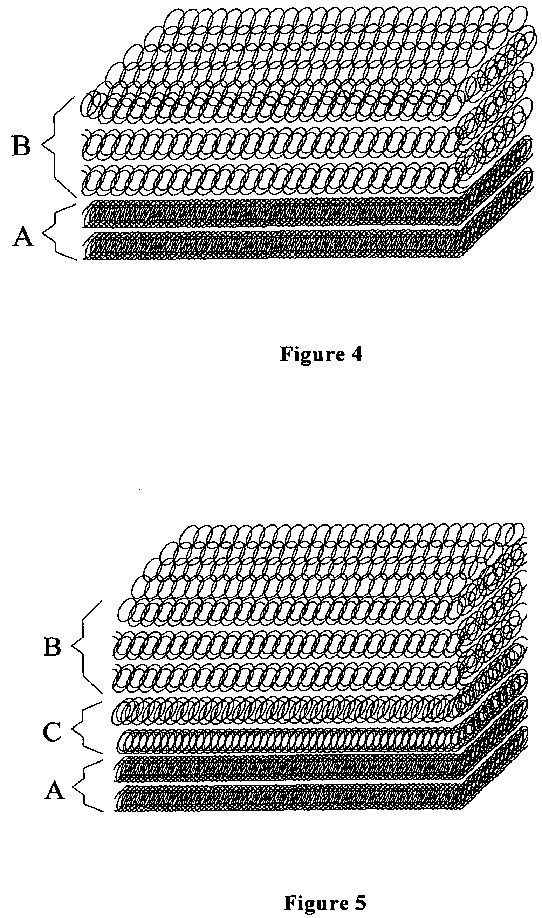

In practice, the above procedure can be repeated, so as to form the hydrophobic layer and/or the hydrophilic layer, as shown in Figure 3 and Figure 4.

Figure 3 gives an artificial dura mater with three hydrophobic layers, and the strength of the artificial dura mater is similar to the human's dura mater. As the layers are formed by the hydrophobic materials, they are not good for the migration and attachment of cell. Together with the fact that the materials are safe, poison-free, and absorbable to human body, they reach the goal of anti-adhesion.

Figure 4 gives an artificial dura mater consisting of two hydrophobic layers (A) and three hydrophilic layers (B). When the dura mater is transplanted into brain, the anti-adhesive hydrophobic layers (A) are set near to the brain surface, while the hydrophilic layers (B) are set far from the brain surface, providing the subtle nanofibrous scaffold for the adhesion, migration, proliferation, and differentiation of cell. As the hydrophilic layers adopt the hydrophilic material that offers good bio-compatibility, and the pore size of the layers are larger, it is good for the migration of stem cell and fibroblast, and consequently is favorable for the growth autologous dura mater.

Once the electrospun layers are made, they will be dried in an oven in natural manner, dependent on the solution component. When the solvent of solution is the volatile, organic solvent, such as the hexafluoro isopropyl alcohol, the procedure for dryness can be omitted since the solvent has completely volatilized while the spinning thread 2 drops to the receiving device 4 alongside the voltage difference.

As the hydrophobic layer is very different from the hydrophilic layer in the terms of affinity to water, the structural stability is not easy to be kept in the application. To solve such problem, the affinity can be increased between the two, or the transition layer can be applied. The affinity of transition layer to water should be gradually stepped up from the hydrophobic layer to the hydrophilic layer. In practice, one or more polymers and corresponding solvent can be determined based on the requirement for the affinity to water. The solvent is put into the sprayer. Also, as per the above-said method, a transition layer can be added. The parameters for the process to form the transition layer are set in this way. The velocity of micro-injection pump is 0.1-5.0 ml per hour. The voltage of high voltage generator is 5-40KV. The receiving distance is 5.0-30.0 cm.

The artificial dura mater with transition layer is illustrated in Figure 5. In the figure, the hydrophobic layers are two, and the hydrophilic layers are three. Between the hydrophobic layers (A) and hydrophilic layers (B), there is two transition layers (C). Between the two transition layers, the one close to hydrophobic layers (A) is weaker than the other close to the hydrophilic layers, in terms of hydrophile.

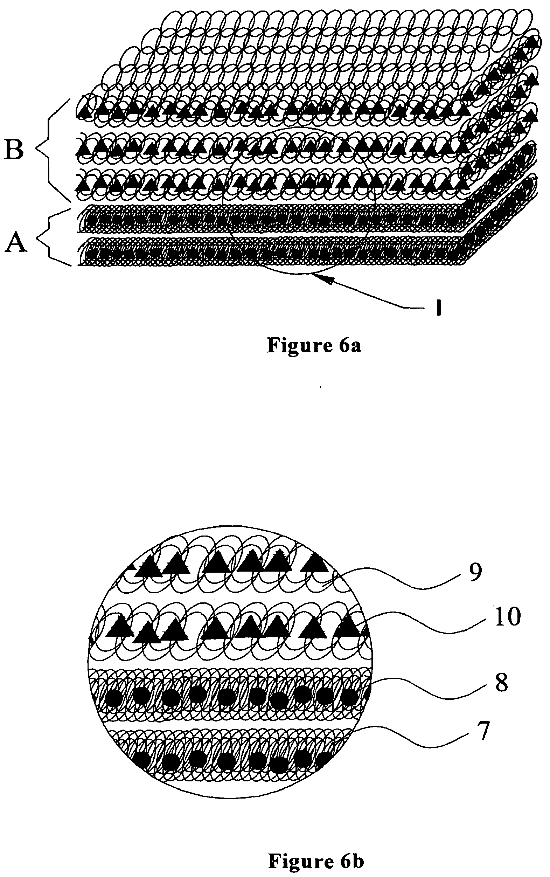

Furthermore, to realize the addition of cytokine and/or medicine into the artificial dura mater, the blending can be adopted. In details, the cytokine and/or medicine can be added to the hydrophobic, hydrophilic, and transition layer, or any two or all of them. The cytokine and/or medicine should be put into the corresponding solutions. After that, the electrospinning is made and the cytokine and/or medicine will be blended into the spinning thread 2 as the spinning threads are formed. The membrane structure will be formed on the receiving device 4. Likewise, this procedure can be repeated. The cytokine and/or medicine added each time can be the same or different. The acquired artificial dura mater is shown in Figure 6. In the Figure 6A, the hydrophobic layer is two-layer structure and the hydrophilic layer is three-layer structure. In the Figure 6B, the hydrophobic spinning thread 7 contains the medicine 8, and the hydrophilic spinning thread 9 is blended with the cytokine 10.

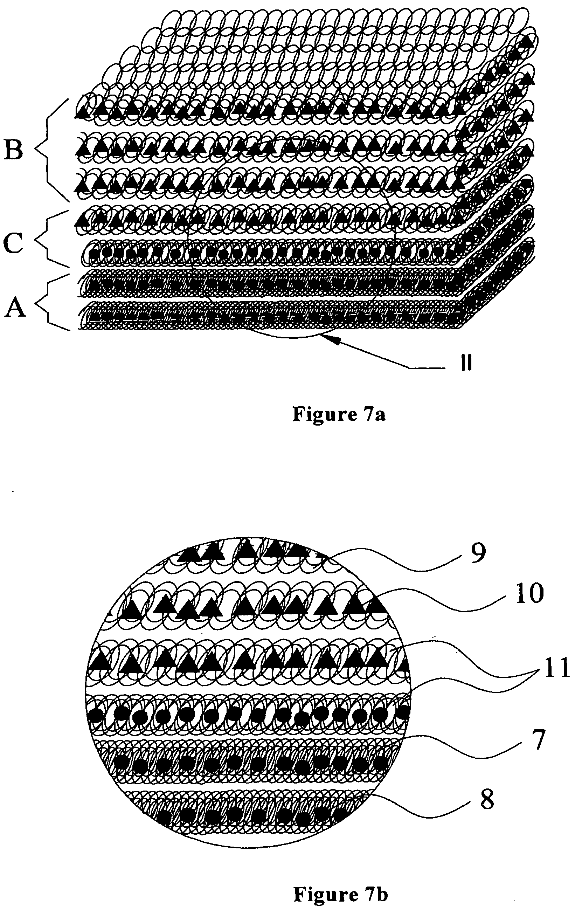

Figure 7A has two transition layers C, based on the Figure 6. The layer of two transition layers, if close to the hydrophobic layer A, its affinity to water is less than that of another layer, which is close to the hydrophilic layer B. From Figure 7B, the hydrophobic spinning thread 7 is blended with medicine 8, and the hydrophilic spinning thread 9 is blended with the cytokine 10, the spinning thread of two transition layers 11 is blended with medicine 8 and cytokine 9.

Beside, the invention provides a process that combines the electrospinning and bio-printing to make the artificial dura mater. The bio-printing is an emerging technology, and just have been developed and applied in biomedical fields in recent years. This technology utilizes the special cell solution or the biologically active solution as the "bio-ink", and gives planned and precise printing on the specific substrate (termed as "bio-paper") that can be degraded in the human body. After the printing, the bio-papers will be stacked in certain sequence. As the printing technology is used, the bio-ink consisting of the cell and/or cytokine can be precisely placed to the designed areas. The bio-papers, if stacked in particular way, will form the three dimensional structure.

The specific implementation is given in Figure 2. Based on the device given in Figure 1, the bio-printer head 5 is set, and such a head can be obtained by modifying the present commercial inkjet printer according to the method, for example, disclosed in patent US 7051654. The head contains the cytokine and/or medicine. The printing manner and printing position can be set through the computer program in advance. The specific printing procedures can base on the current technologies.

In accordance with one embodiment of the invention, the cytokine and/or medicine can be enclosed in the hydrogel. The hydrogel solution can be the one formed by the polysaccharide polymer, polypeptide polymer or synthetic, hydrophilic polymer. The said polysaccharide polymer includes but is not limited to the starch, cellulose, sodium alginate, hyaluronic acid and itosan. The said polypeptide polymer includes but is not limited to the collagen, poly-L-lysine, and PLGA. The said synthetic, hydrophilic polymer includes but is not limited to crylic acid, polymethacrylic acid, polyacrylamide, and N-isopropyl acrylamide. The hydrogel should be liquid in normal circumstances, and become jelly at certain temperature or conditions. Therefore, the hydrogel possesses good adhesion, which can make the cytokine and/or medicine evenly or definitely distribute on the electrostatic spinning layer.

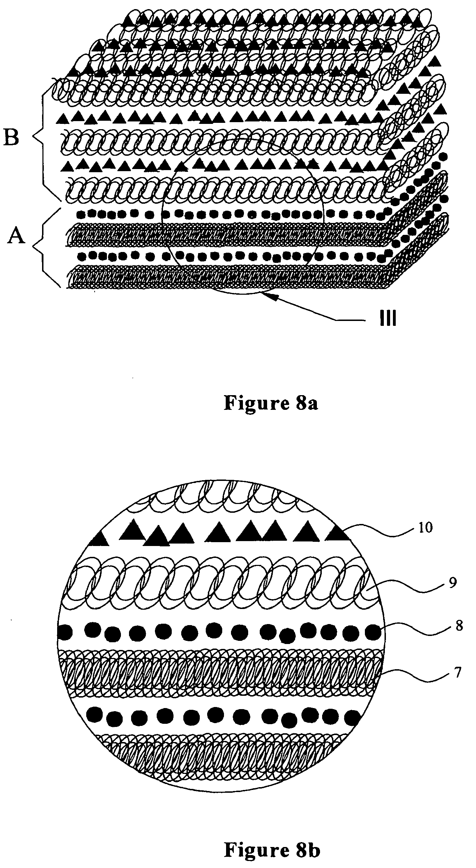

The procedures of bio-printing with hydrogel are as follows. 1) Put the cytokine and/or medicine prepared and blended with liquid hydrogel into the bio-printer head 5. 2) Print on the electrospun layer formed with jet printer as per the preset program, while based on the choice of hydrogel, apply proper conditions to make the hydrogel quickly become gel/jelly that, by offering good adhesion, adhere the cytokine and/or medicine enclosed to the electrospun layers. 3) Set even and uniform distribution of bio-ink and form the artificial dura mater as shown in Figure 8A, in which, each of hydrophobic layers is printed with a layer of medicine and each of hydrophilic layers is printed with a layer of cytokine. As shown in enlarged Figure 8B, the film from bio-printing is different from blending. The cytokine and/or medicine are applied on the surface of layers that is formed by the hydrophobic spinning thread 7 and/or the hydrophilic spinning thread 9. When such dura mater is transplanted into human body, the cytokine and/or medicine can be evenly released. 4) If necessary, set the concentrated distribution on certain points and make the cytokine and/or medicine printed into particular areas. When such dura mater is transplanted to the human body, the cytokine and/or medicine can be mostly released to the wanted specific areas.

The solidification of some hydrogels needs the cross-linking agent to assist. In this case, the bio-printing in the utilization of hydrogel has the following procedures. 1) Put a certain amount of cross-linking agent into the vessel 6. Once the electrospinning starts, the receiving device 4, moving along with the axis or between left and right, will contact the agent, and the formed electrospun layers will fasten (fix) the molecules of the agent. 2) Make the bio-printing as given above. When the liquid hydrogel inside the head 5 contacts the cross-linking agent on the electrospun layers, the hydrogel quickly turns to gel/jelly and enables the cytokine and/or medicine enclosed to attach to the electrospun layers. The selection of cross-linking agent depends on the kind of hydrogel. When the hydrogel is sodium alginate, the cross-linking agent is calcium chloride. When the hydrogel is fibrinogen, the agent is thrombin.

According to the one embodiment of the invention, the said solution of hydrophobic polymer can choose the hydrophobic polylactic acid and ε-caprolactone, and the ratio of two can be 50:50, 30:70 or 70:30. As a copolymer, its number-average molecular weight is 150,000-500,000, and it can be dissolved to the hexafluoro isopropyl alcohol or dichloromethane. When the blending is needed, the hydrophobic solution can be added with 0.01-3% antibiotic solution and/or with 0.001-3% medicine for hemostasis and anti-adhesion. Together with the solution of polylactic acid and ε-caprolactone, the final solution will be obtained.

According to another embodiment of the invention, the solution of hydrophilic polymer can be the hydrophilic polyurethane plus natural gelatin or chondroitine sulfate or polyethylene glycol. The weight ratio is 20-80:80-20. The spinning solution accounts for 3-15% of total weight. In blending, the solution of hydrophilic polymer can be added with the solution of basic fibroblast factor, and make the concentration of cytokine reach up to 0.001-0.5%.

The medicine added in the blending or bio-printing can choose, in accordance with the actual situation, antibiotic or drug for hemostasis or anti-adhesion. In the transplantation of meninges due to tumor excision, the drugs for chemotherapy of brain tumor can be added.

The antibiotic includes but is not limited to the cephalosporin, ampicillin, spiramycin, sulfonamides, and quinolones. The first choice is ceftriaxone sodium. As the meninges operation often need to open the skull and at present, the intracranial infection is often of bacteria, such as staphylococcus aureus, streptococcus, pneumococcus, escherichia coli, salmonella and pseudomonas aeruginosa. The commonest virus is staphylococcus aureus. According to the clinical reports, the ceftriaxone sodium offers better therapy effect.

The anti-tumor medicine includes but is not limited to nimustine, semustine, liposome doxorubicin, dactinomycin D, and vincristine. The vincristine is first choice.

The medicine for hemostasis or anti-adhesion can speed up the healing of wound and prevent the occurrence of adhesion. Such medicine includes but is not limited to the hemostasis factor (which makes the material own the function of hemostasis), the inhibitor of collagen synthase (such as tranilast and alamast, which inhibit the revival of collagen), the anti-coagulation drug (such as dicoumarolum, ehparin sodium, and hirudin), the anti-inflammatory drug (such as promethazine, dexamethasone, hydrocortisonum, prednisolone, ibuprofen, and oxyphenbutazone), the calcium channel blocker (such as diltiazem hydrochloric, nifedipine, and verapamil hydrochloride), the cell growth inhibitor (such as fluorouracil), the hydrolase (such as hyaluronidase, streptokinase, urokinase, pepsin, and tPA), and the oxidation reductant (such as methylene blue).

According to an embodiment of the invention, in the blending, the electrospinning solution is added with the cytokine and/or medicine in this formula: the basic fibroblast growth factor accounting for 0.001-0.05% of electrospinning solution weight, the ampicillin accounting for 3% of electrospinning solution weight, the hemostasis factor accounting for 0.001-0.05% of electrospinning solution weight. In the meninges restoration operation due to brain tumor, the nimustine accounting for 0.01-5% of the weight can be added.

The obtained artificial dura mater should be rinsed, sterilized, packaged and stored.

### Example 1

The polylactic acid and ε-caprolactone, in the weight ratio of 50:50 and with number-average molecular weight of 260,000, are dissolved into the hydrophobic electrospinning solution with the use of the solvent of hexafluoro isopropyl alcohol, and are put into the sprayer of electrospinning. The micro-injection pump is used to adjust the output velocity of the hydrophobic solution to be 5 ml per hour; the high voltage generator is used to adjust the output voltage to be 30KV; and the receiving distance of receiving device to be 20 cm. The fiber is received in the form of membrane structure and the hydrophobic electrospun layer in the average diameter of 300 nm is formed. After the receiving process is completed, the spinning device is closed.

The artificial dura mater acquired is rinsed for five times with ethyl alcohol and distilled water. The dura mater, then, is packaged in vacuum after the freeze dry. After the sterilization with 25kGy cobalt-60, the dura mater is stored at minus 20°C.

### Example 2

The manufacturing method is the same as the example 1.

The hydrophilic solution for electrospinning is the poly ethanedioic acid and chondroitine sulfate, in the mass ratio of 70:30 and the mass fraction of spinning liquid is 9%.

The electrospinning device is activated, and the hydrophilic electrospun layer is made on the hydrophobic layer already formed in Embodiment 1. The receiving distance is 11 cm, the voltage is 20KV, and the acquired hydrophilic layer has the fiber in the average diameter of 10µm.

The rinse and store are the same as those in Embodiment 1.

### Example 3

The hydrophobic electrospun layer is made in the same manner with that in Embodiment 1.

The transition layer adopts the polymer solution of polyurethane and hyaluronic acid in the mass ratio of 70:30 and has the mass fraction at 10%. The spinning is activated, and the receiving distance is 11cm, voltage is 20KV, and the fiber's average diameter is 5µm. On the hydrophobic layer, the transition layer is made.

Then, on the transition layer, the hydrophilic layer is spun, in the same manner as that in Example 4. At final, the spinning is stopped.

The rinse and store are the same as them in Example 1.

### Example 4

(1) Manufacturing the hydrophobic electrospun layer: Choose the solvent of hydrophobic polycaprolactone and chloroform or methyl alcohol in the ratio of 1:1. Add with ceftriaxone sodium, at the concentration of 1%. Get the uniform solution.
   Add the above solution into the sprayer for electrostatic spinning, adjust the velocity of micro-injection pump to be 0.8 ml per hour, the voltage of high voltage generator to be 12KV, and the receiving distance of receiving device to be 15 cm, and obtain the fiber in membrane structure. The diameter of acquired hydrophobic electrospun layer is 600 nanometers.
   Close the spinning device.
(2) Manufacturing the hydrophilic electrospun layer: Adopt the hydrophilic silk protein and natural gelatin in the ratio of 20-80:80-20 and have the mass fraction of spinning liquid at 9%.

Prepare the solution of basic fibroblast factor. Mix the solution with the above-said electrospinning liquid evenly, and have the final concentration of cytokine to be 0.001%, the receiving distance to be 10 cm, and the voltage to be 20KV. Start the electrospinning, and have the hydrophilic layer on the hydrophobic layer formed already. The average diameter of fiber from the hydrophilic layer is in the level of micron.

The rinse and store are the same as them in Example 1.

### Example 5

(1) Manufacturing the hydrophobic electrospun layer: Select the solvent of hydrophobic polycaprolactone and chloroform or methyl alcohol in ratio of 1:1. Blend with vincristine at the concentration of 100ng/ml, and get the uniform solution.
   Add the above solution into the sprayer for electrospinning, adjust the velocity of micro-injection pump to be 0.8 ml per hour, the voltage of high voltage generator to be 12KV, and the receiving distance of receiving device to be 15 cm, and obtain the fiber in membrane structure. The diameter of acquired hydrophobic electrospun layer is 600 nanometers.
   Close the spinning device.
(2) Manufacturing the transition layer: Choose polyurethane and hyaluronic acid in the mass ratio of 70:30. Have the mass fraction of spinning liquid at 10%. Blend with ampicillin at concentration of 3%. Get the uniform solution.
   Start the spinning. Spin the transition layer on the hydrophobic layer already made.
   The receiving distance is 11 cm, the voltage is 20KV, and the average diameter of fiber is 5µm.
   Close the spinning device.
(3) Manufacturing the hydrophilic electrospun layer: Adopt the hydrophilic silk protein and natural gelatin in the ratio of 20-80:80-20 and have the mass fraction of spinning liquid at 9%.The prepared ampicillin solution should be blended with the said spinning liquid, and the final concentration of antibiotic should be 3%.

Activate the spinning device, adjust the receiving distance to be 10cm, and spin the hydrophilic layer on the transition layer already made. The average diameter of fiber should be in the level of micron.

The rinse and store are the same as them in Example 1.

### Example 6

### (1) Manufacturing the hydrophobic electrospun layer with printed cell:

The hydrophobic solution should be the hydrophobic polylactic acid and ε-caprolactone, in the ratio of 50:50, as copolymer. Its number-average molecular weight is 260000 and it can be dissolved into the hexafluoro isopropyl alcohol.

The cross-linking agent should choose 0.1 M calcium chloride solution.

The hydrogel containing the cytokine adopts the solution of hemostasis factor alginate. The hemostasis factor has a percentage of 10pprn in mass in the cytokine alginate solution.

The solution with 0.1 M calcium chloride should be placed into the cell petri dish in the diameter of 150 mm. The receiver share by the spinning device and printer should be placed into the petri dish. In the forming of electrospun layer, the receiving device should contact with the vessels in petri dish. The print head should be fixed due under the electrospinning needle inside the spinning device box, and functions to print on specific area with hemostasis factor. The prepared cytokine alginate solution should be put into the cartridge of jet printer. In this embodiment, the cartridge is HP51626A.

The hydrophobic electrospinning solution should be poured into the sprayer for spinning. After that, the velocity of micro-injection pump should be adjusted to be 5 ml per hour, the voltage of high voltage generator to be 30KV, and the receiving distance of receiving device to be 20 cm. The fiber should be received in membrane structure. The spinning lasts for 20 minutes before the spinning device is closed.

The hydrogel solution containing the cytokine should be printed to the said nano bionic scaffold with jet printer. Once the hydrogel is solidified, the hydrophobic electrospun layer with printed cell is acquired.

### (2) Manufacturing the hydrophilic electrospun layer with printed cell:

The electrospinning solution, the hydrogel solution with medicine and the cross-linking agent solution should be prepared.

Polyglycol as a hydrophilic material and chondroitine sulfate in the mass ratio of 70:30 are chosen. The spinning liquid has the mass fraction of 9%. The cross-linking agent solution is the 0.1 M calcium chloride solution.

The hydrogel solution containing the cytokine is the alginate solution with basic fibroblast factor. The said basic alginate solution has the basic fibroblast factor at the concentration of 100 ppm.

The parameters are set in this way. The velocity of micro-injection pump should be adjusted to be 0.8 ml per hour, the voltage of high voltage generator to be 20KV, and the receiving distance of receiving device to be 11 cm. Other elements are the same as those in first procedure. After the hydrophilic fiber is received in membrane structure, the hydrogel solution containing the basic fibroblast factor is printed to the nano bionic scaffold. Once the hydrogel is solidified, the hydrophilic electrospun layer with printed cell is acquired.

The rinse and store are the same as those in Example 1.

### Example 7

(1) The manufacturing of hydrophobic electrospun layer with printed cell is the same as in Example 6.
(2) Manufacturing the transition layer with printed cell
   The electrospinning liquid of transition layer is the polyurethane and the hyaluronic acid in the mass ratio of 70:30. The mass fraction of spinning liquid is 10%. The blended ampicillin is in the concentration of 3%.
   The cross-linking agent solution is the 0.1 M calcium chloride solution.
   The hydrogel solution with cytokine is the hemostasis factor alginate solution, and the mass percentage of the hemostasis factor is 10ppm.
   The parameters are adjusted in this way. The velocity of micro-injection pump is 4 ml per hour. The voltage of high voltage generator is 20KV. The receiving distance of receiving device is 11 cm. The else are the same as the above procedure. The fiber should be received in membrane structure. The hydrogel solution containing the cytokine will be printed to the transition layer. After the hydrogel is solidified, the I hydrophobic electrospun layer with printed cell is obtained.
(3) The manufacturing of hydrophilic electrospun layer with printed cell is the same as in Example 6.

The rinse and store are the same as those in Example 1.

### Experiment example 1

The dura mater obtained from Example 1 is applied to dog for the animal study, while the control is the commercialized, clinically applicable material for the repair of meninges. Three healthy dogs are chosen, either male or female, in the weight between 10 and 15 kg, and under observation for two to three months. The dogs chosen to use in the experiment are under general anesthesia. Their skulls are opened at both left and right sides. Their dura maters are surgically removed, and the defect of dura maters and injury of brain tissue appear. Then, the dura maters obtained from the Embodiment 1 and the control, respectively, are implanted for repairing the dural defects at the left and right sides of the dog's brain. After operation, the dogs are feed normally as usual and observed periodically. The specimen is taken from the implanted area at the end of each observation period. To retrieve the samples, these dogs are sacrificed by administration of an overdose of intravenous pentobarbital followed by potassium chloride. The skulls are exposed surgically and the implants and surrounding tissue are taken out. The retrieved samples are inspected carefully with details including the surface of implant e, the surrounding tissue, cyst, callosity, as well as the adhesion between the inner surface of the implant and brain tissue. The specimen is fixed and treated with 10%, embedded with paraffin, and cut into 3-5µm sections. Histological staining such as HE staining is applied with these samples.

Several days after implantation, the three dogs recover well, and incisions are healed well with no obvious secretion. The dogs eat and drink normally as well as their outdoor activities are normal without any obvious movement malfunction. Three months post-implantation, the three dogs are sacrifice used the method mentioned above. After sacrifice, taking the surgical site as center, the specimen is cut from the one centimeter more than the operation site, and it includes the repair material, the surrounding dura mater and part of brain tissue. After the specimen is taken, the skull and meninges are separated. It is found that the meninges transplanted in the experiment are formed completely, the site to transplant the material has been replaced by the fibrous tissue, the transplanted artificial dura mater has connected tightly with the native meninges without border, the internal surface of the newly-formed meninges at transplantation site is free from adhesion with the brain tissue, and the surface of corresponding brain tissue is smooth and free from adhesion to the implant. While, in the implanted site of the control samples, the transplanted material not yet to be degraded and a few of adhesion at the transplantation site in the internal surface of meninges exist.

### Experiment example 2

The dura mater manufactured in the Example 2 is now applied for the dog experiment. The five dogs are in weight of 15-20 kg, 1.5-2-year-old, and either male or female. They are in general anesthesia through the intramuscular injection of ketamine. After anesthesia and shave, the animals are placed on the operation table in lateroabdominal position. The disinfection is made with 2% iodine and 75% alcohol. The animals' heads are put in the middle and open surgically in lengthwise direction. Stripper is used to separate the periosteum of the skull, and the two top skull plateaus are exposed. The high-speed driller is used to open the skull. The two skull windows at vertex are formed, and the two rectangular dura maters in size of 3cmx3cm at the top of head are cut off with the scissors. Finally the top dura mater defects are made for the following implantation with artificial dural mater and control. On the exposed brain surface, the electrocoagulation is applied to make 6 injury points in the size of 1mmx1mm. Then, the artificial dura mater manufactured in Embodiment is trimmed to the same shape and size as those of the dural defect and loaded into the defect. The hydrophobic layer is toward the brain surface, and the sutures is made with the 4/0 thread and in the interval of 4mm, to repair the dural defect of dogs. The round needle and 4/0 thread are used for suture of muscle. The commercialized, clinically applicable, animal material based dural repair product is applied as a control. After operation, the dogs are feed normally as usual and observed periodically. The animals recover well, and incisions are healed well. No obvious leakage of cerebrospinal fluid or occurrence of epilepsy is found. The dogs eat and drink normally as well as their outdoor activities are normal without any obvious movement malfunction, and they survive to the expected longevity.

Eighteen months post-implantation, the dogs are sacrificed and the specimen is taken at the surgical site, and it includes the artificial dura mater, the surrounding dura mater, and part of the surrounding brain tissue. After carefully observe the specimen, we can see that the connection between the artificial dura mater and native dura mater is tight and smooth, without clear boundary, healed well, besides, and with thread seen only. The native dura mater does not show any hyperaemia, hemorrhage or other rejection reaction. While, the results with the control show that the implant material is not yet degraded, and at the implanted site, the inner surface of meninges adheres to brain tissue to some degree.

### Experiment example 3

The artificial dura mater obtained from Example 3 is now under the experiment with New Zealand rabbit.

The animals under experiment are opened in the skulls, and the defect of dura mater and injury of brain tissue are made surgically. Then, the artificial dura mater is used to repair the defect. After the operation, the rabbits are feed normally as usual and observed periodically. These animals recover well. Eighteen months post-implantation, the rabbits are sacrificed and the specimen is taken at the surgical site. The specimen includes the artificial dura mater, surrounding dura mater and part of the surrounding brain tissue. When observe the specimen carefully, it is seen that the epithelial cells cover the inner surface of the dura mater; under the epithelium fibrous tissue is formed, fibroblast progenitor cells proliferate, and collagen fibers are produced. All of these result in the formation of new vascularized tissues, in-growth of native dura mater, degradation of the implant material, deduction of total mass of the implant, and generation of rich capillary networks. In the interface of old and new tissues, no neutrophil, lymphocyte or other cell reaction for inflammation is found, and no cyst wall is formed. The arachnoid mater and brain tissue are normal.

### Experiment example 4

The dura mater obtained from Example 4 is now applied to the dog experiment, and the method is the same as Experiment example 2.

Fifteen months post-implantation, the dogs are sacrificed and the specimen is taken at the surgical site. The specimen includes the artificial dura mater, surrounding dura mater, and part of the surrounding brain tissue. After carefully observe the specimen, it is seen that the connection between the artificial dura mater and native dura mater is tight and smooth, without clear boundary, completed cured, and with thread seen only. The native dura mater does not show hyperaemia, hemorrhage or other rejection reaction.

### Experiment example 5

The dura mater obtained from Example 5 is now applied to the New Zealand rabbit experiment, and the control is the commercialized, clinically applicable, animal materials based dura mater product as the repair material.

The method is the same as Experiment example 3. Fifteen months post-implantation, the rabbits are sacrificed and the specimen is taken at the surgical site. When observe the specimen carefully, it is seen that the epithelial cells cover the inner surface of the dura mater; under the epithelium fibrous tissue is formed, fibroblast progenitor cells proliferate, and collagen fibers are produced. All of these result in the formation of new vascularized tissues, in-growth of native dura mater, degradation of the implant material, deduction of total mass of the implant, and generation of rich capillary networks. In the interface of old and new tissues, no neutrophil, lymphocyte or other cell reaction for inflammation is found, and no cyst wall is formed. The arachnoid mater and brain tissue are normal. While, the results with the control show that the implant material is not yet degraded, and at the implanted site, the inner surface of meninges adheres to brain tissue to some degree.

### Experiment example 6

The dura mater obtained from Example 6 is now applied to the dog experiment, and the method is the same as Experiment example 2.

Twelve months post-implantation, the dogs are sacrificed and the specimen is taken at the surgical site.. The specimen includes the artificial dura mater, surrounding dura mater, and part of the surrounding brain tissue. When observe the specimen carefully, it is seen that the connection between the artificial dura mater and native dura mater is tight and smooth, without clear boundary, completed cured, and with thread seen only. The native dura mater does not show hyperaemia, hemorrhage or other rejection reaction.

### Experiment example 7

The dura mater obtained from Example 7 is now applied to the New Zealand rabbit experiment.

The method is the same as Experiment example 3. Twelve months post-implantation, the rabbits are sacrificed and the specimen is taken at the surgical site. When observe the specimen carefully, it is seen that the epithelial cells cover the inner surface of the dura mater; under the epithelium fibrous tissue is formed, fibroblast progenitor cells proliferate, and collagen fibers are produced. All of these result in the formation of new vascularized tissues, in-growth of native dura mater, degradation of the implant material, deduction of total mass of the implant, and generation of rich capillary networks. In the interface of old and new tissues, no neutrophil, lymphocyte or other cell reaction for inflammation is found, and no cyst wall is formed. The arachnoid mater and brain tissue are normal. Notwithstanding the embodiments have been given and described, the person skilled in the field can, without deviation from the principle and theory, can make the variation, modification, replacement and change of these embodiments, as given in the invention scope and right claims.

## Claims

1. An artificial dura mater for use in repair of defective dural tissue, in which the artificial dura mater is made of electrospun layers prepared by electrospinning technology, said electrospun layers consisting of at least one hydrophobic electrospun layer (A) for positioning next to the surface of a brain; said hydrophobic electrospun layer having fibres with a diameter of 50-1000 nanometres, and pores with a size of less than 3 micrometres.

2. The artificial dura mater for use in repair of defective dural tissue of claim 1, wherein said electrospun layer is prepared with one or more hydrophobic polymers by a method of electrospinning, wherein a hydrophobic polymer is selected from the group consisting of hydrophobic aliphatic polyester, polyetherester, polyorthoester, polyurethane, polyanhydride, polyphosphazene, polyamino acid and copolymers and mixtures thereof.

3. The artificial dura mater for use in repair of defective dural tissue of claim 2, wherein said hydrophobic aliphatic polyester is at least one selected from the group consisting of polylactic acid, polyglycolide, polycaprolactone and polyhydroxybutyrate.

4. The artificial dura mater for use in repair of defective dural tissue of claim 2, wherein said polyetherester is at least one selected from the group consisting of polydioxanone, glycol/lactic acid copolymer, or glycol/butylene terephthalate copolymer.

5. The artificial dura mater for use in repair of defective dural tissue of claim 2, wherein said polyanhydride is poly(sebacic acid-hexadecanedoic acid anhydride).

6. The artificial dura mater for use in repair of defective dural tissue of claim 1, wherein at least one hydrophilic electrospun layer (B) is further provided on said hydrophobic electrospun layer for positioning away from the surface of the brain.

7. The artificial dura mater for use in repair of defective dural tissue of claim 6, wherein said hydrophilic electrospun layer comprises fibers with a diameter of 5-200 micrometres and pores with a size of 20-200 micrometres, and said hydrophilic electrospun layer is prepared by a method of electrospinning with one or more hydrophilic polymers selected from the group consisting of chondroitine sulfate, heparin, agar, glucan, algin, modified cellulose, alginate, starch, cellulose, gelatin, fibrinogen, silk protein, elastin-mimicry peptide polymer, collagen, chitosan, modified chitosan, hydrophilic polyurethane, polyethylene glycol, polymethylmethacrylate, PHBV, PHBHHx, polyvinyl alcohol, polylactide, and mixtures thereof.

8. The artificial dura mater for use in repair of defective dural tissue of claim 6, wherein a transition layer (C) is disposed between the hydrophobic and hydrophilic electrospun layers and said transition layer is prepared, by a method of electrospinning, with one or more polymers, and the hydrophilicity of said transition layer increases from said hydrophobic electrospun layer to said hydrophilic electrospun layer.

9. The artificial dura mater for use in repair of defective dural tissue of claim 6, further comprising a cytokine (10) and/or a medicine (8) added or adhered to said hydrophobic electrospun layer.

10. The artificial dura mater for use in repair of defective dural tissue of claim 7, further comprising a cytokine (10) and/or a medicine (8) added or adhered to said hydrophobic electrospun layer and/or said hydrophilic electrospun layer.

11. The artificial dura mater for use in repair of defective dural tissue of claim 8, further comprising a cytokine (10) and/or a medicine (8) added or adhered to said hydrophobic electrospun layer and/or said hydrophilic electrospun layer and/or said transition layer.

12. The artificial dura mater for use in repair of defective dural tissue of any one of claims 9 to 11, wherein said cytokine is selected from the group consisting of interteukin, colony stimulating factor, tumour necrosis factor, platelet derived growth factor, basic fibroblast factor, and combinations thereof, and said medicine is one or more selected from the group consisting of an antibiotic, a hemostat, an anti-adhesion agent, and an anti-tumour drug.

13. A method of preparing the artificial dura mater for use in repair of defective dural tissue according to any one of claims 1 to 12, the method of preparing comprising the steps of
a) dissolving a hydrophobic polymer into a solvent to obtain hydrophobic electrospinning solution, wherein said hydrophobic polymer is selected from the group consisting of hydrophobic aliphatic polyester, polyetherester, polyorthoester, polyurethane, polyanhydride, polyphosphazene, polyamino acid and copolymers or mixtures thereof,
b) producing a film-like hydrophobic electrospun layer having fibers of a diameter of 50-1000 nanometres and pores with a size of less than 3 micrometres by electrospinning from said hydrophobic electrospinning solution to obtain the artificial dura mater.

14. The method of preparing the artificial dura mater for use in repair of defective dural tissue of claim 13, wherein at step b), said electrospinning is performed with a micro-injection pump operated at a velocity of 0.1-5.0 millilitres per hour, and a high voltage generator operated at a voltage of 5-40 kilovolts and with a receiving distance of 5.0-30.0 centimetres.

15. The method of preparing the artificial dura mater for use in repair of defective dural tissue of claim 13, further comprising steps of forming a hydrophilic electrospun layer on the hydrophobic electrospun layer:
a') dissolving the hydrophilic polymer into a solvent to obtain the electrospinning solution with hydrophilic polymer, said hydrophilic polymer being selected from the group consisting of the following substances: chondroitine sulfate, heparin, agar, glucan, algin, modified cellulose, alginate, starch, cellulose, gelatin, fibrinogen, silk protein, elastin-mimicry peptide polymer, collagen, chitosan, modified chitosan, hydrophilic polyurethane, polyethylene glycol, polymethylmethacrylate, PHBV, PHBHHx, polyvinyl alcohol and polylactide and mixtures thereof;
b') placing the electrospinning solution on the hydrophobic electrospun layer by electrospinning to form the hydrophilic electrospun layer.

16. The method of preparing the artificial dura mater for use in repair of defective dural tissue of claim 15, wherein said hydrophilic electrospun layer comprises fibers with a diameter of 5-200 micrometres and pores with a size of 20-200 micrometres, and wherein at step b'), said electrospinning is performed with a micro-injection pump operated at 0.1-20.0 millilitres per hour and a high voltage generator operated at a voltage of 10-40 kilovolts and with a receiving distance of 5.0-30.0 centimetres.

17. The method of preparing the artificial dura mater for use in repair of defective dural tissue of claim 15, further comprising the step of forming a transition layer between the hydrophobic and hydrophilic electrospun layers by electrospinning an electrospinning solution containing one or more polymers before the hydrophilic electrospun layer is prepared, and such that the transition layer's hydrophilicity will gradually increase from the hydrophobic electrospun layer to the hydrophilic electrospun layer.

18. The method of preparing the artificial dura mater for use in repair of defective dural tissue of claim 13 or claim 16, further comprising the step that a cytokine (10) and/or a medicine (8) are distributed on said hydrophobic electrospun layer and/or said hydrophilic electrospun layer by bio-printing technique, wherein said bio-printing technique comprises the steps of:
a") admixing a hydrogel solution with a cytokine and/or a medicine to form a solution; and
b") printing said mixed solution onto said hydrophobic and/or hydrophilic electrospun layers using bio-printing technology.

## Patentansprüche

1. Eine künstliche Dura mater zum Einsatz bei der Heilung von beschädigtem Duralgewebe. Die künstliche Dura Mater besteht aus elektrogesponnenen Schichten, die mithilfe des Elektrospinnverfahrens gefertigt werden. Mindestens eine der elektrogesponnenen Schichten ist eine hydrophobe elektrogesponnene Schicht (A), die zur Oberfläche des Gehirns hin aufgebracht wird. Besagte hydrophobe elektrogesponnene Schicht besteht aus Fasern mit einem Durchmesser von 50-1000 Nanometern und weniger als 3 Mikrometern großen Poren.

2. Die unter Patentanspruch 1 genannte künstliche Dura mater zum Einsatz bei der Heilung von beschädigtem Duralgewebe, deren besagte elektrogesponnene Schicht mithilfe eines Elektrospinnverfahrens aus einem oder mehreren hydrophoben Polymeren gefertigt wird. Das hydrophobe Polymer wird aus folgender Gruppe ausgewählt: hydrophober aliphatischer Polyester, Polyetherester, Polyorthoester, Polyurethan, Polyanhydrid, Polyphosphazen, Polyaminosäure und Copolymere sowie Kombinationen aus diesen Stoffen.

3. Die unter Patentanspruch 2 genannte künstliche Dura mater zum Einsatz bei der Heilung von beschädigtem Duralgewebe, wobei der besagte hydrophobe aliphatische Polyester aus folgender Gruppe ausgewählt wird (mindestens ein Stoff): Polymilchsäure, Polyglykolid, Polycaprolacton und Polyhydroxybuttersäure.

4. Die unter Patentanspruch 2 genannte künstliche Dura mater zum Einsatz bei der Heilung von beschädigtem Duralgewebe, wobei besagter Polyetherester aus folgender Gruppe ausgewählt wird (mindestens ein Stoff): Polydioxanon, Copolymer aus Glykol- und Milchsäure oder Copolymer aus Glykol- und Butylenterephthalat.

5. Die unter Patentanspruch 2 genannte künstliche Dura mater zum Einsatz bei der Heilung von beschädigtem Duralgewebe, wobei besagtes Polyanhydrid Poly(Sebacinsäure-Hexadecandisäure-Anhydrid) ist.

6. Die unter Patentanspruch 1 genannte künstliche Dura mater zum Einsatz bei der Heilung von beschädigtem Duralgewebe, wobei mindestens eine hydrophile elektrogesponnene Schicht (B) auf die besagte hydrophobe elektrogesponnene Schicht aufgebracht wird und von der Gehirnoberfläche weg zeigt.

7. Die unter Patentanspruch 6 genannte künstliche Dura mater zum Einsatz bei der Heilung von beschädigtem Duralgewebe, deren besagte hydrophile elektrogesponnene Schicht aus Fasern mit einem Durchmesser von 5-200 Mikrometern und 20-200 Mikrometer großen Poren besteht. Besagte hydrophile elektrogesponnene Schicht wird mithilfe eines Elektrospinnverfahrens aus einem oder mehreren hydrophilen Polymeren gefertigt, die aus folgender Gruppe ausgewählt werden: Chondroitinsulfat, Heparin, Agar, Glucan, Algin, modifizierte Cellulose, Alginat, Stärke, Cellulose, Gelatine, Fibrinogen, Seidenprotein, Polymer aus Elastin-Mimikry-Peptiden, Kollagen, Chitosan, modifiziertes Chitosan, hydrophiles Polyurethan, Polyethylenglykol, Polymethylmethacrylat, PHBV, PHBHHx, Polyvinylalkohol, Polylactid sowie Kombinationen aus diesen Stoffen.

8. Die unter Patentanspruch 6 genannte künstliche Dura mater zum Einsatz bei der Heilung von beschädigtem Duralgewebe, deren hydrophobe und hydrophile elektrogesponnene Schichten mit einer Zwischenschicht (C) verbunden sind. Besagte Zwischenschicht wird mithilfe eines Elektrospinnverfahrens aus einem oder mehreren Polymeren gefertigt und die Hydrophilie besagter Zwischenschicht nimmt von der besagten hydrophoben elektrogesponnenen Schicht hin zur besagten hydrophilen elektrogesponnenen Schicht zu.

9. Die unter Patentanspruch 6 genannte künstliche Dura mater zum Einsatz bei der Heilung von beschädigtem Duralgewebe, die darüber hinaus ein Zytokin (10) und/oder ein Medikament (8) enthält, das der besagten hydrophoben elektrogesponnenen Schicht beigemischt oder darauf aufgebracht wird.

10. Die unter Patentanspruch 7 genannte künstliche Dura mater zum Einsatz bei der Heilung von beschädigtem Duralgewebe, die darüber hinaus ein Zytokin (10) und/oder ein Medikament (8) enthält, das der besagten hydrophoben elektrogesponnenen Schicht und/oder der besagten hydrophilen elektrogesponnenen Schicht beigemischt oder darauf aufgebracht wird.

11. Die unter Patentanspruch 8 genannte künstliche Dura mater zum Einsatz bei der Heilung von beschädigtem Duralgewebe, die darüber hinaus ein Zytokin (10) und/oder ein Medikament (8) enthält, das der besagten hydrophoben elektrogesponnenen Schicht und/oder der besagten hydrophilen elektrogesponnenen Schicht und/oder der besagten Zwischenschicht beigemischt oder darauf aufgebracht wird.

12. Die unter einem der Patentansprüche 9 bis 11 genannte künstliche Dura mater zum Einsatz bei der Heilung von beschädigtem Duralgewebe, wobei besagtes Zytokin aus folgender Gruppe ausgewählt wird: Interleukin, koloniestimulierender Faktor, Tumor-Nekrose-Faktor, thrombozytärer Wachstumsfaktor, basaler Fibroblastenfaktor sowie Kombinationen der Genannten. Besagtes Medikament wird aus folgender Gruppe ausgewählt (ein oder mehrere Medikamente): ein Antibiotikum, ein Hämostatikum, ein Antiadhäsionswirkstoff und ein Tumormedikament.

13. Ein Herstellungsverfahren für die unter einem der Patentansprüche 1 bis 12 genannte künstliche Dura mater zum Einsatz bei der Heilung von beschädigtem Duralgewebe. Das Herstellungsverfahren umfasst folgende Schritte:
a) Auflösung eines hydrophoben Polymers zu einem Lösungsmittel, um eine hydrophobe Lösung für das Elektrospinnverfahren zu erhalten. Besagtes hydrophobes Polymer wird dabei aus folgender Gruppe ausgewählt: hydrophober aliphatischer Polyester, Polyetherester, Polyorthoester, Polyurethan, Polyanhydrid, Polyphosphazen, Polyaminosäure und Copolymere, oder Kombinationen aus diesen Stoffen;
b) Herstellung einer filmartigen, hydrophoben elektrogesponnenen Schicht aus Fasern mit einem Durchmesser von 50-1000 Nanometern und weniger als 3 Mikrometer großen Poren mithilfe des Elektrospinnverfahrens, wobei besagte hydrophobe Elektrospinn-Lösung verwendet und somit die künstliche Dura mater gefertigt wird.

14. Das unter Patentanspruch 13 genannte Herstellungsverfahren für die künstliche Dura mater zum Einsatz bei der Heilung von beschädigtem Duralgewebe, wobei das unter Schritt b) genannte Elektrospinnverfahren mittels einer Mikroinjektionspumpe mit einem Durchfluss von 0,1-5,0 Millilitern pro Stunde und einem Hochspannungsgenerator mit einer Spannung von 5-40 Kilovolt und einem Empfangsabstand von 5,0-30,0 Zentimetern durchgeführt wird.

15. Das unter Patentanspruch 13 genannte Herstellungsverfahren für die künstliche Dura mater zum Einsatz bei der Heilung von beschädigtem Duralgewebe, das darüber hinaus folgende Schritte zur Bildung einer hydrophilen elektrogesponnenen Schicht auf der hydrophoben elektrogesponnenen Schicht umfasst:
a. Auflösung des hydrophilen Polymers zu einem Lösungsmittel, um die Lösung für das Elektrospinnverfahren mit hydrophilem Polymer zu erhalten. Besagtes hydrophiles Polymer wird dabei aus folgender Gruppe ausgewählt: Chondroitinsulfat, Heparin, Agar, Glucan, Algin, modifizierte Cellulose, Alginat, Stärke, Cellulose, Gelatine, Fibrinogen, Seidenprotein, Polymer aus Elastin-Mimikry-Peptiden, Kollagen, Chitosan, modifiziertes Chitosan, hydrophiles Polyurethan, Polyethylenglykol, Polymethylmethacrylat, PHBV, PHBHHx, Polyvinylalkohol und Polylactid sowie Kombinationen aus diesen Stoffen;
b. Aufbringen der Elektrospinn-Lösung mittels Elektrospinnverfahren auf die hydrophobe elektrogesponnene Schicht, um die hydrophile elektrogesponnene Schicht herauszubilden.

16. Das unter Patentanspruch 15 genannte Herstellungsverfahren für die künstliche Dura mater zum Einsatz bei der Heilung von beschädigtem Duralgewebe, deren besagte hydrophile elektrogesponnene Schicht aus Fasern mit einem Durchmesser von 5-200 Mikrometern und 20-200 Mikrometer großen Poren besteht. Besagtes unter Schritt b') genanntes Elektrospinnverfahren wird mittels einer Mikroinjektionspumpe mit einem Durchfluss von 0,1-20,0 Millilitern pro Stunde und einem Hochspannungsgenerator mit einer Spannung von 10-40 Kilovolt und einem Empfangsabstand von 5,0-30,0 Zentimetern durchgeführt.

17. Das unter Patentanspruch 15 genannte Herstellungsverfahren für die künstliche Dura mater zum Einsatz bei der Heilung von beschädigtem Duralgewebe, das darüber hinaus einen Fertigungsschritt zur Bildung einer Zwischenschicht zwischen den hydrophoben und hydrophilen elektrogesponnenen Schichten umfasst. Bei diesem mittels Elektrospinnverfahren durchgeführten Schritt wird vor der Herausbildung der hydrophilen elektrogesponnenen Schicht eine Elektrospinn-Lösung aus einem oder mehreren Polymeren aufgebracht, und zwar so, dass die Hydrophilie der Zwischenschicht von der hydrophoben elektrogesponnenen Schicht hin zur hydrophilen elektrogesponnenen Schicht schrittweise zunimmt.

18. Das unter den Patentansprüchen 13 oder 16 genannte Herstellungsverfahren für die künstliche Dura mater zum Einsatz bei der Heilung von beschädigtem Duralgewebe, das darüber hinaus einen Fertigungsschritt umfasst, in dem mittels eines Bioprinting-Verfahrens ein Zytokin (10) und/oder ein Medikament (8) auf besagte hydrophobe elektrogesponnene Schicht und/oder besagte hydrophile elektrogesponnene Schicht aufgebracht wird. Besagtes Bioprinting-Verfahren umfasst dabei folgende Schritte:
a) Mischung einer Hydrogel-Lösung mit einem Zytokin und/oder einem Medikament, um eine Lösung zu erhalten; und
b) Druck dieser gemischten Lösung auf besagte hydrophobe und/oder hydrophile elektrogesponnene Schicht mithilfe der Bioprinting-Technologie.

## Revendications

1. Une dure-mère artificielle pour l'utilisation dans la réparation du tissu dural défectueux, où la dure-mère artificielle est composée de couches électrofilées préparées grâce à la technologie d'électrofilage, lesdites couches électrofilées se composant d'au moins une couche hydrophobe électrofilée (A) pour le positionnement à côté de la surface d'un cerveau ; ladite couche hydrophobe électrofilée possédant des fibres d'un diamètre de 50 à 1000 nanomètres, et des pores d'une taille de moins de 3 micromètres.

2. La dure-mère artificielle pour l'utilisation dans la réparation du tissu dural défectueux de la revendication 1, où ladite couche électrofilée est préparée avec un ou plusieurs polymères hydrophobes grâce à la méthode d'électrofilage, où un polymère hydrophobe est sélectionné parmi le groupe composé de polyester aliphatique hydrophobe, de polyétherester, de polyorthoester, de polyuréthane, de polyanhydride, de polyphosphazène, de polyamino-acide et de copolymères ainsi que de mélanges de ceux-ci.

3. La dure-mère artificielle pour l'utilisation dans la réparation du tissu dural défectueux de la revendication 2, où ledit polyester aliphatique hydrophobe est au moins un sélectionné parmi le groupe composé d'acide polylactique, de polyglycolide, de polycaprolactone et de polyhydroxybutyrate.

4. La dure-mère artificielle pour l'utilisation dans la réparation du tissu dural défectueux de la revendication 2, où ledit polyétherester est au moins un sélectionné parmi le groupe composé de polydioxanone, de copolymère d'acide lactique / de glycol, ou de copolymère de téréphtalate de butylène/glycol.

5. La dure-mère artificielle pour l'utilisation dans la réparation du tissu dural défectueux de la revendication 2, où ledit polyanhydride est poly(acide sébacique - acide hexadécanédioïque anhydride).

6. La dure-mère artificielle pour l'utilisation dans la réparation du tissu dural défectueux de la revendication 1, où au moins une couche hydrophile électrofilée (B) est également fournie sur ladite couche hydrophobe électrofilée pour le positionnement à l'écart de la surface du cerveau.

7. La dure-mère artificielle pour l'utilisation dans la réparation du tissu dural défectueux de la revendication 6, où ladite couche hydrophile électrofilée comporte des fibres d'un diamètre de 5 à 200 micromètres et des pores d'une taille de 20 à 200 micromètres, et ladite couche hydrophile électrofilée est préparée grâce à une méthode d'électrofilage avec un ou plusieurs polymères hydrophiles sélectionnés parmi le groupe composé de sulfate de chondroïtine, d'héparine, d'agar, de glucane, d'algine, de cellulose modifiée, d'alginate, d'amidon, de cellulose, de gélatine, de fibrinogène, de protéine de soie, de polymère de peptide imitation d'élastine, de collagène, de chitosan, de chitosan modifié, de polyuréthane hydrophile, de polyéthylène glycol, de polyméthylméthacrylate, de PHBV, de PHBHHx, d'alcool polyvinylique, de polylactide, ainsi que de mélanges de ceux-ci.

8. La dure-mère artificielle pour l'utilisation dans la réparation du tissu dural défectueux de la revendication 6, où une couche de transition (C) est placée entre les couches hydrophobe et hydrophile électrofilées et ladite couche de transition est préparée, grâce à une méthode d'électrofilage, avec un ou plusieurs polymères, et l'hydrophilicité de ladite couche de transition augmente de ladite couche hydrophobe électrofilée à ladite couche hydrophile électrofilée.

9. La dure-mère artificielle pour l'utilisation dans la réparation du tissu dural défectueux de la revendication 6, comprenant également une cytokine (10) et/ou un médicament (8) ajoutés ou adhérant à ladite couche hydrophobe électrofilée.

10. La dure-mère artificielle pour l'utilisation dans la réparation du tissu dural défectueux de la revendication 7, comprenant également une cytokine (10) et/ou un médicament (8) ajoutés ou adhérant à ladite couche hydrophobe électrofilée et/ou à ladite couche hydrophile électrofilée.

11. La dure-mère artificielle pour l'utilisation dans la réparation du tissu dural défectueux de la revendication 8, comprenant également une cytokine (10) et/ou un médicament (8) ajoutés ou adhérant à ladite couche hydrophobe électrofilée et/ou à ladite couche hydrophile électrofilée et/ou à ladite couche de transition.

12. La dure-mère artificielle pour l'utilisation dans la réparation du tissu dural défectueux des revendications 9 à 11, où ladite cytokine est sélectionnée parmi le groupe composé d'interleukine, d'un facteur stimulant les colonies, d'un facteur de nécrose des tumeurs, d'un facteur de croissance dérivé des plaquettes, d'un facteur de croissance basique des fibroblastes, et de combinaisons de ceux-ci, et où le ou lesdits médicaments sont sélectionnés parmi le groupe composé d'un antibiotique, d'un produit hémostatique, d'un agent anti-adhésif, et d'un traitement anti-cancéreux.

13. Une méthode de préparation de la dure-mère artificielle pour l'utilisation dans la réparation du tissu dural défectueux conformément à une quelconque des revendications 1 à 12, la méthode de préparation comportant les étapes de :
a) dissolution d'un polymère hydrophobe dans un solvant pour obtenir une solution hydrophobe d'électrofilage, où ledit polymère hydrophobe est sélectionné parmi le groupe composé de polyester aliphatique hydrophobe, de polyétherester, de polyorthoester, de polyuréthane, de polyanhydride, de polyphosphazène, de polyamino-acide et de copolymères ou de mélanges de ceux-ci,
b) production d'une couche hydrophobe électrofilée agissant comme un film avec des fibres d'un diamètre de 50 à 1 000 nanomètres et des pores d'une taille de moins de 3 micromètres en électrofilant à partir de ladite solution hydrophobe d'électrofilage afin d'obtenir la dure-mère artificielle.

14. La méthode de préparation de la dure-mère artificielle pour l'utilisation dans la réparation du tissu dural défectueux de la revendication 13, où à l'étape b), ledit électrofilage est effectué avec une pompe de micro-injection fonctionnant à une vitesse de 0,1 à 5,0 millilitres par heure, et avec un générateur haute tension fonctionnant à une tension de 5 à 40 kilovolts et avec une distance de réception de 5,0 à 30,0 centimètres.

15. La méthode de préparation de la dure-mère artificielle pour l'utilisation dans la réparation du tissu dural défectueux de la revendication 13, comportant également les étapes de formation d'une couche hydrophile électrofilée sur la couche hydrophobe électrofilée :
a) dissolution du polymère hydrophile dans un solvant afin d'obtenir la solution d'électrofilage avec le polymère hydrophile, ledit polymère hydrophile étant sélectionné parmi le groupe composé des substances suivantes : sulfate de chondroïtine, héparine, agar, glucane, algine, cellulose modifiée, alginate, amidon, cellulose, gélatine, fibrinogène, protéine de soie, polymère de peptide imitation d'élastine, collagène, chitosan, chitosan modifié, polyuréthane hydrophile, polyéthylène glycol, polyméthylméthacrylate, PHBV, PHBHHx, alcool polyvinylique et polylactide et mélanges de ceux-ci ;
b) positionnement de la solution d'électrofilage sur la couche hydrophobe électrofilée en électrofilant afin de former la couche hydrophile électrofilée.

16. La méthode de préparation de la dure-mère artificielle pour l'utilisation dans la réparation du tissu dural défectueux de la revendication 15, où ladite couche hydrophile électrofilée est composée de fibres d'un diamètre de 5 à 200 micromètres et de pores d'une taille de 20 à 200 micromètres, et où lors de l'étape b), ledit électrofilage est effectué avec une pompe de micro-injection fonctionnant à une vitesse de 0,1 à 20,0 millilitres par heure et avec un générateur haute tension fonctionnant à une tension de 10 à 40 kilovolts et avec une distance de réception de 5,0 à 30,0 centimètres.

17. La méthode de préparation de la dure-mère artificielle pour l'utilisation dans la réparation du tissu dural défectueux de la revendication 15, comportant également les étapes de formation d'une couche de transition entre les couches hydrophile et hydrophobe électrofilées en électrofilant une solution d'électrofilage contenant un ou plusieurs polymères avant la préparation de la couche hydrophile électrofilée, et de manière à ce que l'hydrophilicité de la couche de transition augmente graduellement de la couche hydrophobe électrofilée à la couche hydrophile électrofilée.

18. La méthode de préparation de la dure-mère artificielle pour l'utilisation dans la réparation du tissu dural défectueux de la revendication 13 ou 16, comportant également l'étape de distribution d'une cytokine (10) et/ou d'un médicament (8) sur ladite couche hydrophobe électrofilée et/ou ladite couche hydrophile électrofilée grâce à une technique de bio-impression, où ladite technique de bio-impression comporte les étapes de :
a) mélange d'une solution d'hydrogel avec une cytokine et/ou un médicament pour former une solution ; et
b) impression de ladite solution mélangée sur lesdites couches hydrophobe et/ou hydrophile électrofilées grâce à une technologie de bio-impression.
